# EUROPEAN PATENT APPLICATION

(11) **EP 1 566 636 A1**
(43) Date of publication of application: **24.08.2005**
(21) Application number: 04004094.1
(22) Date of filing: 23.02.2004
(51) Int. Cl.: G01N 33/50, A61K 39/395, A61K 38/17, A61P 25/00, A61K 31/7088

(54) **Use of Tweak modulators and inhibitors for the treatment of neurological conditions**

(71) Applicant: AXARON Bioscience AG, 69120 Heidelberg (DE)
(72) Inventor: Schneider, Armin, 69118 Heidelberg (DE); Rossner, Moritz, 37077 Göttingen (DE); Vogt, Gerhart, 69126 Heidelberg (DE); Schwaninger, Markus, 68165 Mannheim (DE)
(74) Representative: Isenbruck, Günter

(57) **Abstract**

The invention relates to the use of substances that modulate or inhibit the expression or activity of TWEAK in neural cells, for the manufacture of a medicament for the treatment and/or prophylaxis of neurological and/or psychiatric conditions.

The invention furthermore relates to the use of substances that modulate or inhibit the expression or activity of the TWEAK receptor, or that modulate or inhibit the intracellular signalling of the TWEAK receptor, for the same purpose.

In addition, the invention encompasses a method for the identification of substances that modulate or inhibit the expression or activity of TWEAK.

## Description

The object of the present invention is the use of substances that modulate or inhibit the expression or activity of TWEAK in neural cells, for the manufacture of a medicament for the treatment and/or prophylaxis of neurological and/or psychiatric conditions.

The invention furthermore relates to the use of substances, that modulate or inhibit the expression or activity of the TWEAK receptor, or that modulate or inhibit the intracellular signalling of the TWEAK receptor, for the same purpose.

In addition, the invention encompasses a method for the identification of substances that modulate or inhibit the expression or activity of TWEAK.

TNF-like weak inducer of apoptosis (TWEAK), is a member of the tumor necrosis factor (TNF) superfamily. The members of this family are known to be involved in apoptosis, proliferation, migration, and inflammation. Most of the tumor necrosis factors are type II transmembrane proteins, but the extracellular domains of TNFs can be cleaved by specific metalloproteases, resulting in soluble cytokines. These soluble cytokines may then interact with specific receptors (Orlinick et al. (1998), *Cell Signal.* 10(8):543-51).

The human TWEAK gene is encoded by 750 base pairs and located on chromosome 17. It is translated to a 250 amino acid protein, with an apparent molecular weight of ~30 kDa. The sequence homology between mouse, rat and human is rather high (93% mouse and human; 90% rat and human; in the receptor binding domain), suggesting the conservation of structure and function (Chicheportiche et al. (1997), *J Biol Chem.,* 272(51):32401-10).

Unprocessed TWEAK has one transmembrane domain and is mainly localized to the outer cell membrane. After processing, the secreted and soluble form of the protein is about 17 kDa, and it assembles to the TNF-ligand-typical trimeric active form. This TWEAK trimer binds to a specific receptor, that was identified by expression cloning as fibroblast growth factor-inducible gene (Fn14). Studies confirmed that TWEAK binds Fn14 (the TWEAK receptor), leading to the activation of intracellular signalling cascades involving NF-kB activation, thereby mediating pro-inflammatory and cell death effects (Han S et al. (2003), Biochem Biophys Res Commun. 2003,13;305(4):789-96).

Several studies showed that TWEAK induces apoptosis in tumor cells and that it acts in a proinflammatory way by upregulation of adhesion molecules and the secretion of cytokines (Wiley et al. (2003), *Cytokine Growth Factor Rev*., 14(3-4):241-9. review). It was also shown to promote proliferation of endothelial cells and angiogenesis. Especially in combination with bFGF it was described to stimulate neovascularization.

TWEAK transcripts are abundant and found in many tissues. TWEAK that is secreted by leukocytes, was reported to infiltrate the inflamed brain and act on astrocytes. These astrocytes bind the ligand, but are resistant to TWEAK mediated apoptosis. It was observed that the cells secreted high amounts of IL-8 and IL-6, suggesting that the TWEAK plays a role in brain inflammation (Saas P et al. (2000), *Glia.* 32(1): 102-7).

For a variety of neurological illnesses there is no adequate method of treatment available: Stroke is the third-leading cause of death, and the main cause of disability in the western world. It presents a large socioeconomic burden. The etiology can be either ischemic (in the majority of cases) or hemorrhagic. The cause of ischemic stroke is often embolic, or thrombotic. So far, there is no effective treatment for the majority of stroke patients. The only clinically proven drugs so far are tissue plasminogen activator (TPA) and Aspirin. After massive cell death in the immediate infarct core due to lack of glucose and oxygen, the infarct area expands for days, owing to secondary mechanisms such as glutamate excitotoxicity, apoptotic mechanisms, and generation of free radicals.

Amyotrophic lateral sclerosis (ALS; Lou-Gehrig's disease; Charcot's disease) is a neurodegenerative disorder with an annual incidence of 0.4 to 1.76 per 100.000 population (Adams et al., *Principles of Neurology,* 6^{th} ed., New York, pp 1090-1095). It is the most common form of motor neuron disease with typical manifestations of generalized fasciculations, progressive atrophy and weakness of the skeletal muscles, spasticity and pyramidal tract signs, dysarthria, dysphagia, and dyspnea. The pathology consists principally in loss of nerve cells in the anterior horn of the spinal cord and motor nuclei of the lower brainstem, but can also include the first order motor neurons in the cortex. Pathogenesis of this devastating disease is still largely unknown, although the role of superoxide-dismutase (SOD1) mutants in familial cases has been worked out quite well, which invokes an oxidative stress hypothesis. So far, more than 90 mutations in the SOD1 protein have been described, that can cause ALS (Cleveland and Rothstein (2001), *Nat Rev* *Neurosci,* 2, 806-19). Also, a role for neurofilaments in this disease was shown. Excitotoxicity, a mechanism evoked by an excess glutamate stimulation is also an important factor, exemplified by the beneficial role of Riluzole in human patients. Most convincingly shown in the SOD1 mutants, activation of caspases and apoptosis seems to be the common final pathway in ALS (Ishigaki, et al. (2002), *J Neurochem,* 82, 576-84., Li, et al. (2000), *Science,* 288, 335-9). Therefore, it seems that ALS also falls into the same general pathogenetic pattern that is also operative in other neurodegenerative diseases and stroke, e.g. glutamate involvement, oxidative stress, and programmed cell death.

Parkinson's disease is the most frequent movement disorder, with approximately 1 million patients in North America; about 1 percent of the population over the age of 65 years is affected. The core symptoms of the disease are rigor, tremor and akinesia (Adams et al., Principles of Neurology, 6^{th} ed., New York, pp 1090-1095). The etiology of Parkinson's disease is not known. Nevertheless, a significant body of biochemical data from human brain autopsy studies and from animal models points to an ongoing process of oxidative stress in the substantia nigra, which could initiate dopaminergic neurodegeneration. Oxidative stress, as induced by the neurotoxins 6-hydroxydopamine and MPTP (N-methyl-4-phenyl-1,2,3,6- tetrahydropyridine), has been used in animal models to investigate the process of neurodegeneration. Although a symptomatic therapy exists (e.g. L-DOPA plus a decarboxylase inhibitor; bromocriptine, pergolide as dopamin agonists; and anticholinergic agents such as trihexyphenidyl (artane)), there is a clear need for a causative therapy, e.g. a neuroprotective therapy, that really halts the disease progress. These animal models have been used to test the efficacy of radical scavengers, iron chelators, dopamine agonists, nitric oxide synthase inhibitors and certain calcium channel antagonists. Apoptotic mechanisms are clearly operative in the animal models as well as in the patient (Mochizuki, et al. (2001), *Proc. Natl. Acad. Sci. USA,* 98, 10918-23, Xu et al. (2002), *Nat. Med.,* 8, 600-6, Viswanath, et al. (2001), *J. Neurosci.,* 21, 9519-28, Hartmann, et al. *(2002), Neurology,* 58, 308-10). This pathophysiology with involvement of oxidative stress and apoptosis also places Parkinson's disease amongst the other neurodegenerative disorders and stroke.

Cerebral ischemia may result from a variety of causes that impair cerebral blood flow (CBF) and lead to deprivation of both oxygen and glucose. Traumatic brain injury (TBI), on the other hand, involves a primary mechanical impact that usually causes skull fracture and abruptly disrupts the brain parenchyma with shearing and tearing of blood vessels and brain tissue. This, in turn, triggers a cascade of events characterized by activation of molecular and cellular responses that lead to secondary injury. The evolution of such secondary damage is an active process in which many biochemical pathways are involved (Leker and Shohami (2002), *Brain Res. Rev.,* 39, 55-73). Many similarities between the harmful pathways that lead to secondary cellular death in the penumbral ischemic zone and in the area exposed to secondary post-traumatic injury have been identified (e.g. excitotoxity by excess glutamate release, nitric oxide, reactive oxygen species, inflammation, and apoptosis (Leker and Shohami *(2002), Brain Res. Rev.,* 39, 55-73)). In addition, early ischemic episodes are reported to occur after traumatic brain injury, adding a component of ischemia to the primary mechanical damage.

Cardiovascular disease is the major cause of death in western industrialized nations. In the United States, there are approximately 1 million deaths each year with nearly 50% of them being sudden and occurring outside the hospital (Zheng, et al. (2001), *Circulation, 104,* 2158-63). Cardio-pulmonary resuscitation (CPR) is attempted in 40-90 of 100,000 inhabitants annually, and restoration of spontaneous circulation (ROSC) is achieved in 25-50% of these patients. However, the hospital discharge rate following successful ROSC is only 2-10% (Bottiger, et al. (1999), *Heart,* 82, 674-9). Therefore, the vast majority of the cardiac arrest victims annually in the United States is not treated successfully. The major reason for the low survival rates after successful CPR, i.e., for postarrest in-hospital mortality, is persistent brain damage. Brain damage following cardiocirculatory arrest is related both to the short period of tolerance to hypoxic stress and to specific reperfusion disorders (Safar (1986), *Circulation,* 74, IV138-53, Hossmann (1993), *Resuscitation, 26,* 225-35). Initially, a higher number of patients can be stabilized hemodynamically after cardiocirculatory arrest; many of them, however, die due to central nervous system injury. The personal, social, and economic consequences of brain damage following cardiac arrest are devastating. One of the most important issues in cardiac arrest and resuscitation ("whole body ischemia and reperfusion") research, therefore, is cerebral resuscitation and postarrest cerebral damage (Safar (1986), *Circulation,* 74, IV138-53, Safar, et al. (2002), *Crit Care Med,* 30, p. 140-4). Presently, it is not possible to decrease the primary damage to neurons that is caused by hypoxia during cardiac arrest by any post-arrest therapeutic measures. Major pathophysiological issues include hypoxia and subsequent necrosis, reperfusion injury with free radical formation and cellular calcium influx, release of excitatory amino acids, cerebral microcirculatory reperfusion disorders, and programmed neuronal death or apoptosis (Safar (1986), *Circulation,* 74, IV138-53, Safar et al. (2002), *Crit Care Med,* 30, 140-4).

Several clinical trials have attempted to improve neurological outcome after cardiac arrest without success. The therapeutic use of barbiturates (to enhance neuroprotection) or the use of calcium channel blockers (to reduce ischemia reperfusion damage) was tested (Group (1986), *Am. J. Emerg. Med.,* 4, 72-86, Group (1986), *N. Engl. J. Med.,* 314, 397-403, Group (1991), *Control Clin. Trials,* 12, 525-45, Group (1991), *N. Engl. J. Med., 324,* 1225-31). To date no specific post-arrest treatment options are available to improve neurological outcome following cardiocirculatory arrest in the clinical setting (with the possible exception of mild hypothermia and thrombolysis where the results of large, randomized, and controlled clinical trials are eagerly awaited (Safar et al (2002), *Crit. Care Med.,* 30, 140-4)). Therefore, an innovative therapy to improve neurological outcome after cardiac arrest is crucial.

Multiple sclerosis is the prototype inflammatory autoimmune disorder of the central nervous system and, with a lifetime risk of one in 400, potentially the most common cause of neurological disability in young adults. Worldwide, there are about 2 - 5 million patients suffering from this disease (Compston and Coles (2002), *Lancet,* 359, 1221-31. ). As with all complex traits, the disorder results from interplay between as yet unidentified environmental factors and susceptibility genes. Together, these factors trigger a cascade of events, involving engagement of the immune system, acute inflammatory injury of axons and glia, recovery of function and structural repair, post-inflammatory gliosis, and neurodegeneration. The sequential involvement of these processes underlies the clinical course characterized by episodes with recovery, episodes leaving persistent deficits, and secondary progression. The aim of treatment is to reduce the frequency, and limit the lasting effects of relapses, relieve symptoms, prevent disability arising from disease progression, and promote tissue repair.

Schizophrenia is one of the most common mental illnesses. About 1 of every 100 people (1% of the population) is affected by schizophrenia. This disorder is found throughout the world and in all races and cultures. Schizophrenia affects men and women in equal numbers, although on average, men appear to develop schizophrenia earlier than women. Generally, men show the first signs of schizophrenia in their mid 20s and women show the first signs in their late 20s. Schizophrenia has a tremendous cost to society, estimated at $32.5 billion per year in the US. Schizophrenia is characterized by several of the following symptoms: delusions, hallucinations, disorganized thinking and speech, negative symptoms (social withdrawal, absence of emotion and expression, reduced energy, motivation and activity), catatonia. The main therapy for schizophrenia is based on neuropleptics, such as chlorpromazine, haloperidol, olanzapine, clozapine, thioridazine, and others. However, neuroleptic treatment often does not reduce all of the symptoms of schizophrenia. Moreover, antipsychotic treatment can have severe side effects, such as tardive dyskinesias. The etiology of schizophrenia is not clear, although there seems to be a strong genetic influence. Recently, it has become clear that schizophrenia has at least some aspects of a neurodegenerative disease. In particular, MR studies have revealed rapid cortical grey matter loss in schizophrenic patients (Thompson, et al. (2001), *Proc Natl Acad Sci U S A,* 98, 11650-5; Cannon, et al. (2002), *Proc Natl Acad Sci U S A,* 99, 3228-33). Therefore, treatment of schizophrenics with neuroprotective medication such as TWEAK inhibition is warranted.

In view of the above, there is a need for medicaments for the treatment of neurological and/or psychiatric conditions, such as neurological diseases that relate to the enhancement of plasticity and functional recovery, or cell-death in the nervous system. In particular, there is a need for treating neurological diseases by providing neuroprotection to the neural cells involved.

These problems are solved by the present invention, the use of a substance that modulates or inhibits the expression or activity of TWEAK and/or the TWEAK receptor, or modulates or inhibits the intracellular signalling of the TWEAK receptor, in neural cells, for the manufacture of a medicament for the treatment and/or prophylaxis of neurological and/or psychiatric conditions. I.e. this results in a method for the for the treatment or prophylaxis or treatment and prophylaxis of neurological or psychiatric conditions using a substance, that that modulates or inhibits the expression or activity of TWEAK or of the TWEAK receptor, or of TWEAK and the TWEAK receptor, or that modulates or inhibits the intracellular signalling of the TWEAK receptor, in neural cells.

The term "substance" is to be understood broadly and means, in a general manner, all the material means which directly or indirectly bring about the desired effect. By way of example substances according to the invention can be nucleic acids or proteins, natural or artificial binding partners of TWEAK or the TWEAK receptor, antibodies, antagonists of the TWEAK receptor, peptidomimetics of a TWEAK receptor antagonist, antisense nucleic acids, aptamers, natural or artificial transcription factors, nucleic acid constructs, vectors or low molecular weight compounds.

"Modulate" or "modulation" means the increase or decrease of at least one essential property, or of the expression of TWEAK. "Inhibit" or "inhibition" comprises the partial or essentially complete suppression or blocking of expression or activity based on different cell-biological mechanisms. With a statistical probability a significant difference is observable compared to an untreated cell in both cases.

The term "activity" means according to the invention the ability of TWEAK to bind to and/or activate its receptor.

By "treatment" is meant the slowing, interrupting, arresting or stopping of the progression of the disease or condition and does not necessarily require the complete elimination of all disease symptoms and signs. "Prophylaxis" is understood to be any degree of inhibition of the time of onset or severity of signs or symptoms of the disease or condition, including, but not limited to, the complete prevention of the disease or condition.

To achieve these effects, the substances are administered in a therapeutically effective amount. A therapeutically effective amount of the substances according to the invention results in a beneficial effect for the treated individual. Such an amount can be determined based on age, race, sex, and other factors based on the individual patient. When the factors are administered in combination, they may be premixed prior to administration, administered simultaneously, or administered singly in series. The route of administration can include the typical routes including, for example, orally, subcutaneously, transdermally, rectally, intravenously, intraarterially, by direct injection to the brain, intranasally (by nasal spray) and parenterally.

The skilled person will recognize that a number of different methods are available for influencing TWEAK in the desired manner. In this respect, the methods which are described below are to be understood as being examples.

In a preferred embodiment the substance, which is capable of modulating or inhibiting the expression or activity of TWEAK, its receptor or influence the intracellular signalling from this receptor is an antibody. In the first place, such antibodies themselves constitute compounds, which posses a specific binding affinity for TWEAK, the TWEAK receptor or a protein in the signal transduction pathway triggered by the TWEAK receptor, and/ or is able to modulate at least one essential property of these proteins. The term "antibody" comprises according to the invention polyclonal antibodies as well as monoclonal antibodies, human and humanized antibodies, chimeric antibodies, single chain antibodies, synthetic antibodies, or fragments of these antibodies. Fragments according to the invention are all the truncated or modified antibody fragments which have one or two binding sites which are complementary to the antigen. Preference is given to truncated double-stranded fragments, such as Fv, Fab and F(ab)₂. These fragments can be obtained, for example, either enzymatically, by cleaving of the Fc moiety of the antibodies using enzymes such as papain or pepsin. or by means of chemical oxidation or by means of recombinantly manipulating the antibody genes.

All antibodies can be used in bound or soluble form and may be labelled. Labelling means the direct or indirect linking to detectable substances. Antibodies according to the invention can also be part of fusion proteins. These fusion proteins can be manufactured by enzymatic cleavage, by protein synthesis or by recombination methods known by a person skilled in the art.

Antibodies according to the invention are understood as meaning all the immunoglobulin classes, such as IgM, IgG, IgD, IgE, and IgA or their subclasses such as the IgG subclasses IgG1, IgG2, IgG2a, IgG2b, IgG3 or IgGM, or their mixtures. Particular preference is given to the IgG subtypes IgG17k or IgG2b/k.

Monoclonal antibodies can be obtained by techniques, with which a person skilled in the art is familiar, for example, by means of the hybridoma technique (Köhler and Milstein, *Nature* (1975),256:495-397; US 4 276 110). Polyclonal antibodies are a heterogeneous mixture of antibody molecules that are derived from animal sera, which had been immunized with the antigen. According to the invention antibodies are also polyclonal monospecific antibodies, which are received by purification of the antibodies.

All these antibodies or fragments can be used either on their own or in mixtures.

In a preferred embodiment the substance, which is capable of modulating or inhibiting the expression or activity of TWEAK or its receptor is an antisense nucleotide acid sequence. Antisense nucleotide acid sequences act at the mRNA level, preventing its translation into proteins (Kurreck, *Eur. J Biochem.* (2003), 270:1629-1644).

An "antisense" nucleic acid sequence means a nucleic acid sequence which is entirely or partially complementary to a part of the sense strand of a nucleic acid sequence. The antisense nucleotide acid sequence according to this invention is complementary to the coding region of the TWEAK nucleic acid sequence or fragments thereof, or functional equivalents, or functionally equivalent fragments thereof. Accordingly the antisense nucleotide sequence can be complementary to the TWEAK receptor or to proteins from the respective transduction pathway or to fragments thereof. However, the antisense nucleic acid sequence can also be complementary to the non-coding region or a part thereof. In a preferred embodiment, the antisense nucleic acid is an oligonucleotide having a length of, for example, 15, 20, 25, 30, 35, 40, 45 or 50 nucleotides, preferably of 15, 16, 17, 18, 19 or 20 nucleotides.

An antisense nucleic acid can be prepared chemically and/or enzymatically using methods with which the skilled person is familiar. These antisense ologonucleotides can be modified by analogs with unnatural bases, modified sugars or altered phosphate backbones. Modified nucleotides may increase the biological stability of the antisense nucleic acid or the physical stability of the duplex which is formed between the antisense nucleic acid and the sense nucleic acid. Phosphorothioate derivatives and acridine-substituted nucleotides may be mentioned by way of example (Eckstein, *Antisense Nucleic Acids Drug Dev.* (2000), 10:297-310). Alternatively, an antisense nucleic acid can also be produced biologically, using an expression vector into which the corresponding nucleic acid has been inserted, in the antisense orientation, downstream of a suitable promoter. The skilled person is aware of the fact, that alternatively cDNA can be used as a starting template for appropriate antisense constructs.

Some "second generation" antisense nucleotides cannot induce RNAse H cleavage of the target RNA. In a preferred embodiment the "gapmer technology" is used for these types of modified nucleotides. Gapmers consist of a central stretch of DNA or phosphorothioate DNA monomers sufficient to activate RNase H, and second generation modified nucleotides at each end. Among these may be Peptide nucleic acids, N3'-P5' phosphoroamidates, 2'-Deoxy-2'-fluoro-β-D-arabino nucleic acids, locked nucleic acids, morpholino oligonucleotides, cyclohexene nucleic acids, and tricyclo-DNA (Kurreck, *Eur. JBiochem.* (2003), 270:1628-1644).

Delivery systems, that mediate a highly efficient cellular uptake and protect the antisense nucleotides against nucleic degradation, are known to a person skilled in the art and can be used according to the invention (Hughes et al., (2001) *Drug Discovery Today,* 6:303-315; Liang et al., (2002) *Eur. J. Biochem.,* 269:5753-5758).

Double-stranded RNA-mediated gene interference (RNAi) is a mechanism of gene silencing in which the presence of a double-stranded RNA-Molecule reduces or silences the expression of gene with a common sequence. In a preferred embodiment the substance, which is capable of modulating or inhibiting the expression or activity of TWEAK or its receptor or modulates or inhibits the intracellular signalling of the TWEAK receptor is siRNA. The term "siRNA" as used herein refers to any nucleic acid molecule capable of mediating RNA interference.

Preferably, the dsRNA molecules which are complementary to the target mRNA of the present invention have a length between 10 and 30 base pairs, more preferably, they have a length between 19 and 25 base pairs. The siRNA may be delivered to the target cell by any method known to the one of skilled art. Applicable is, for instance, the delivery by using cationic liposome reagents. It is also conceivable that the siRNA directed against the cytokine mRNA is obtained by using the DNA encoding it. In this case, a DNA construct comprising both a stretch of 19 to 25 nucleotides of the desired cytokine coding region, and the antisense stretch being separated from the sense stretch by a suitable linker which is able to form a hairpin loop, is inserted into a vector. The design of such a construct is further described e.g. in Brummelkamp et al. (Science 2002 Vol. 296, pages 550-553).

In a preferred embodiment of the invention the antisense nucleotide and/or siRNA is provided by a viral vector or a liposome. The selection of a suitable vector and expression control sequences as well as vector construction is known. Examples, of viral vectors include an adenovirus (Acsadi et al., *Hum. Gene Ther.* 7(2): 129-140 (1996); Quantin et al., *PNAS USA* 89(7): 2581-2584 (1992); and Ragot et al., *Nature* 361 (6413): 647-650 (1993)), an adeno-associated viral vector (Rabinowitz et al., *Curr. Opin. Biotechnol.* 9(5): 470-475 (1998)), a retroviral vector (Federico, *Curr. Opin. Biotechnol.* 10(5): 448-453 (1999)), a *Herpes simplex* viral vector (Latchman, *Gene* 264(1): 1-9 (2001)), a lentiviral vector, a *Sindbis* viral vector, or a Semliki forest viral vector.

Suitable vectors are also liposomes containing proteins that will attach to neural cells, e.g., virus epitopes, and contain either nucleic acid encoding GCSF or GMCSF, or protein, or oligonucleotides. An example of such a transfer system is the HVJ-liposome (Kaneda, et al. (2002), *Mol Ther,* 6, 219-26. Kaneda (1999), *Mol Membr Biol,* 16, 119-22.). Preferably, the isolated and purified nucleic acid encoding and expressing the protein or polypeptide is operably linked to a promoter that is suitable for expression in neural cells. These and other suitable vectors are reviewed in Kay et al., *Nature Medicine* 7: 33-40 (2001); Somia et al., *Nature Reviews* 1: 91-99 (2000); and van Deutekom et al., *Neuromuscular Disorders* 8: 135-148 (1998).

Suitable promoters for operable linkage to the isolated and purified nucleic acid are known in the art. Preferably, the isolated and purified nucleic acid encoding the polypeptide is operably linked to a promoter selected from the group consisting of the muscle creatine kinase (MCK) promoter (Jaynes et al., *Mol. Cell Biol.* 6: 2855-2864 (1986)), the cytomegalovirus (CMV) promoter, a tetracycline/doxycycline-regulatable promoter (Gossen et al., *PNAS USA* 89: 5547-5551 (1992)).

Generally, to ensure effective transfer of the vectors of the present invention, about 1 to about 5,000 copies of the vector are employed per cell to be contacted, based on an approximate number of cells to be contacted in view of the given route of administration, and it is even more preferred that about 3 to about 300 pfu enter each cell. These viral quantities can be varied according to the need and use whether *in vitro* or *in vivo*. The actual dose and schedule can also vary depending on whether the composition is administered in combination with other compositions, e.g., pharmaceutical compositions, or depending on individual differences in pharmacokinetics, drug disposition, and metabolism. Similarly, amounts can vary in *in vitro* applications depending on the particular type of cell or the means by which the vector is transferred.

In a preferred embodiment of the invention the substance, that modulates or inhibits the expression or activity of TWEAK and/or the TWEAK receptor, or modulates or inhibits the intracellular signalling of the TWEAK receptor, in neural cells, is a ribozyme. As used herein "ribozymes" are intended to include RNA molecules that contain antisense sequences for specific recognition, and an RNA-cleaving enzymatic activity. Ribozymes are designed to target the target mRNA through complementary base pair hybridization. After binding to the target, the enzymatic activity of the ribozyme cleaves the target mRNA thus preventing its translation into protein. Two types of ribozymes are particularly useful in this invention, the hammerhead ribozyme (Rossi et al., (1991) *Pharmac. Ther.* 50:245-254) and the hairpin ribozyme (Hampel et al.(1990), *Nucl. Acid. Res.* 18:299-304)

The expression of ribozymes for the purpose of decreasing particular proteins is known to the skilled person. Suitable target sequences and ribozymes can be determined by means of secondary structure calculations of ribozyme and target RNA and also by means of their interaction.

The ribozymes of the invention typically consist of RNA, but such ribozymes may also be composed of chimeric nucleic acid sequences. In addition, the ribozymes may be chemically modified allowing the ribozymes to be more stable and active.

In a preferred embodiment of the invention the substance, that modulates or inhibits the expression or activity of TWEAK and/or the TWEAK receptor, or modulates or inhibits the intracellular signalling of the TWEAK receptor, in neural cells, is an antagonist of the TWEAK receptor. According to the present invention "antagonist" refers to a substance able block a receptor by displacing the physiological active transmitter substances or their analogs, thereby not inducing a physiological reaction and/or signal transduction.

In a preferred embodiment of the invention the antagonist has a molecular weight of less than 2000 g/mol, preferably less than 1000 g/mol, particularly preferably less than 750 g/mol, and most preferably less 500 g/mol.

In a preferred embodiment of the invention the substance, that modulates or inhibits the expression or activity of TWEAK and/or the TWEAK receptor, or modulates or inhibits the intracellular signalling of the TWEAK receptor, in neural cells, is a soluble part of the TWEAK receptor. A soluble part of the TWEAK receptor according to the invention is to be understood as being a part of the TWEAK receptor, or a functional equivalent thereof, that is able to bind and therefore block TWEAK, but that is not capable of inducing a physiological reaction and/or signal transduction. Preferably this is the N-terminal part of the TWEAK receptor, or a mutated form of it. The soluble part of the TWEAK receptor can be manufactured according to methods known by a person skilled in the art, as proteolytic digest or genetic engineering.

Neurological conditions that are to be treated according to the present invention can be generally classified into three classes: those diseases (with pathophysiological mechanisms) with ischemic or hypoxic mechanisms; neurodegenerative diseases; and neurological and psychiatric diseases associated with neural cell death.

Diseases with pathophysiological mechanisms involving ischemia and/or hypoxia can be further subclassified into stroke (as well as hemorrhagic stroke), cerebral ischemia after resuscitation, intrapartal hypoxia, intraoperative hypoxia and/or ischemia and intraocular ischemia and/or hypoxia.

Examples of neurodegenerative diseases include stroke, ALS, Parkinson's disease, cerebral ischemia, multiple sclerosis, schizophrenia, depression, Huntington's disease, trinucleotide repeat disorders, peripheral neuropathies, dementias, and CNS trauma.

Examples of diseases of the nervous system accompanied by neural cell death include stroke, ALS, Parkinson's disease, cerebral ischemia, cerebral ischemia after resuscitation cardiovascular disease, multiple sclerosis, schizophrenia, intrapartal hypoxia, intraoperative hypoxia and/or ischemia and intraocular ischemia and/or hypoxia, depression, Huntington's disease, trinucleotide repeat disorders, peripheral neuropathies, dementias, and CNS trauma.
Also preferred are modifications of the substances according to the invention that increase its ability to cross the blood-brain barrier, or shift its distribution coefficient towards brain tissue. An example for such a modification is the addition of Protein transduction domain (PTD) or TAT sequences (Cao G, et al (2002) *J. Neurosci.* 22:5423-5431; Mi Zet al (2000) *Mol. Ther.* 2:339-347; Morris et al (2001) *Nat Biotechnol* 19:1173-1176; Park et al (2002) *J Gen Virol* 83:1173-1181). These sequences can also be used in mutated forms, and added with additional amino acids at the amino- or carboxyterminus of proteins.

The above-described substances according to the invention can be formulated for medical purposes according to standard procedures available in the art, e.g., a pharmaceutically acceptable carrier (or excipient) can be added. A carrier or excipient can be a solid, semisolid or liquid material which can serve as a vehicle or medium for the active ingredient. The proper form and mode of administration can be selected depending on the particular characteristics of the product selected, the disease, or condition to be treated, the stage of the disease or condition, and other relevant circumstances *(Remington's Pharmaceutical Sciences,* Mack Publishing Co. (1990)). The proportion and nature of the pharmaceutically acceptable carrier or excipient are determined by the solubility and chemical properties of the substance selected the chosen route of administration, and standard pharmaceutical practice. The pharmaceutical preparation may be adapted for oral, parenteral or topical use and may be administered to the patient in the form of tablets, capsules, suppositories, solution, suspensions, or the like.

In a preferred embodiment, the medicament can further comprise one or more additional factors. "Additional factors" according to the invention are any substances that further support the beneficial effect of the medicament. Adding bradykinin, or analogous substances to an intravenous application of any preparation will support its delivery to the brain, or spinal cord (Emerich et al (2001) *Clin Pharmacokinet* 40:105-123; Siegal et al (2002) *Clin Pharmacokinet* 41:171-186). Antiapoptotic agents or agents that facilitate the passage over the blood brain barrier may also be used. A person skilled in the art is familiar with the additional factors that are beneficial for the treatment of the individual illnesses.

In a preferred embodiment the invention relates to a method for identifying a substance, that modulates or inhibits the expression or activity of TWEAK, comprising incubating a neural cell culture under conditions that mimic stroke, contacting the substance with the cells, and comparing the level of TWEAK gene expression of these cells with the level of TWEAK gene expression of cells not contacted with the substance but incubated under the same conditions.

The "conditions that mimic stroke" can be created by e.g. apoptose-inducing substances, the expression of Huntingdon-proteins or oxygen-glucose deprivation. Methods for the measurement of gene expression are well known by a person skilled in the art

The substances identified by these methods can be used for the manufacture of medicaments for the treatment and/or prophylaxis of neurological and/or psychiatric conditions as well.

### The following figures elucidate the invention:

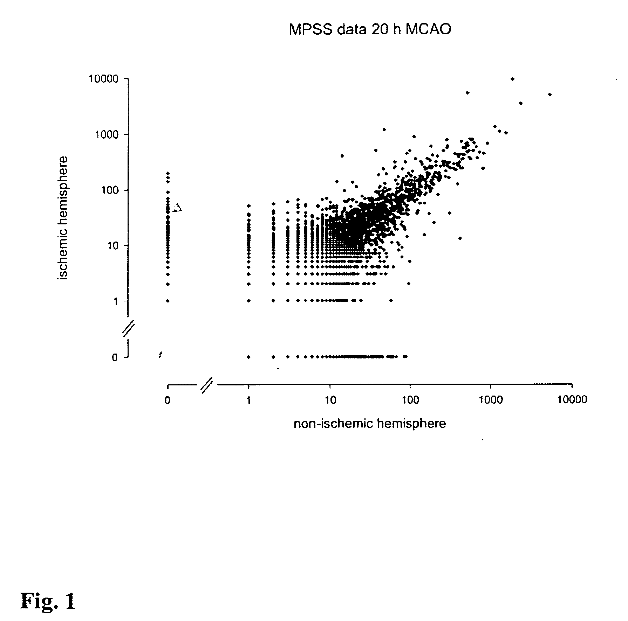

Figure 1 shows the result of the MPSS experiment on the comparison of the left (ischemic; y-axis) to the right (non-ischemic; x-axis) hemisphere. Given are the frequency of occurrences of the signatures identified. A signature for TWEAK ("GATCCCTGTGGATTTTG") was identified at a frequency of 41 in the ischemic hemisphere and 0 in the contralateral, non-ischemic hemisphere (arrow; p = 1.12 10⁻¹²). The total number of signatures sequenced were 174812 for the left hemisphere, and 161809 for the right hemisphere.

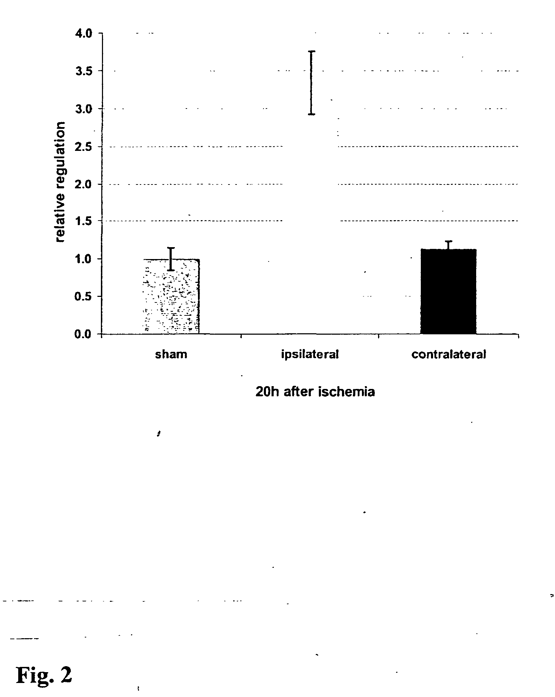

Figure 2 shows the specific upregulation of tweak ipsilateral to the lesion side 20h after focal cerebral ischemia by more than 3-fold, as detected by quantitative PCR. Error bars indicate standard deviations of serial dilution measurements.

### Examples

TWEAK was identified as a upregulated sequence in the MCAO model, a valid stroke model in rodents, by using the MPSS technology. Gene expression changes during cerebral ischemia are of importance for ischemic pathophysiology and stroke outcome. We therefore consider TWEAK an important new pharmacological target in stroke, and other neurological conditions that share central mechanisms with stroke such as neurodegeneration.

### Methods used for the examples

### Ischemic model

The middle cerebral artery occlusion model (MCAO) for stroke was used. Mice were anesthesized using 70% N₂O, 30% O₂, and 1% halothane. The external carotid artery was preparated, and a 5-0 nylon filament inserted. The artery was rotated 180°, and the filament advanced into the internal carotid artery until the middle cerebral artery was reached. The 5-0 nylon filament was blunted at the tip. Successful occlusion was monitored using laser Doppler flowmetry (Perimed, Sweden). 90 min occlusion was followed by 20 h reperfusion. Thereafter, animals were sacrificed by deep anesthesia, and transcardial perfusion with hanks balanced salt solution (HBSS). Hemispheric forebrains (cerebellum, olfactory bulb, and brain stem removed) were further processed for RNA using acidic phenol extraction.

### MPSS expression profiling

For expression profiling, RNA from hemispheres (ipsi- and contralateral) of 6 animals was pooled to reduce the influence of interindividual variation in gene expression. Massively parallel signature sequencing (MPSS) was essentially carried out as described (Brenner et al.,(2000) *Nat. Biotechnol.*,18:630-634) with some modifications. Briefly, in this method RNA is converted into cDNA and the 3'-most DpnII fragments are recovered. After in vitro cloning the cDNA templates are immobilized on separate 5 µm diameter glass beads. Loaded microbeads are placed into a flow cell forming a densely packed monolayer. Short sequences from the free template ends are obtained simultaneously by a fluorescence-based ligation-mediated sequencing method. Obtained signatures (14 bases) are sufficiently long to allow the identification of the vast majority (greater than 95%; compare (Velculescu et al., (1995) *Science* 270, 484-7) of the individual cDNAs. Signatures matching more than one gene (taking into account only Non-EST or EST EMBL database entries) can be detected by clustering all matching sequences excluding putative sequencing errors and sequence polymorphisms.

To prepare cDNA-libraries for the MPSS analysis, 5 µg of total RNA was denatured at 70 °C with 50 pmol of T7-tagged-BsmBI-oligo-dT18V primer (GGC CAG TGA ATT GTA ATA CGA CTC ACT ATA GGG CTG CAT TGA GAC GAT TCT TTT TTT TTT TTT TTT TTV), cooled on ice, reverse transcribed with 200 U of Superscript II at 42 °C for 1 h in 1 reaction buffer, 10 mM dithiothreitol (all reagents Invitrogen, Karlsruhe, Germany) and 0.5 mM each dNTP (Roche Diagnostics, Mannheim, Germany) in 25 µL. Second strand cDNA synthesis was performed by adding 40 U of DNA polymerase I, 2 U of RNase H, 10 U Escherichia coli DNA ligase and 0.5 mM each dNTP in 1 second-strand buffer (Invitrogen) in a final volume of 100 µL for 2 h at 16 °C. RNA was hydrolyzed in the presence of 100 mM NaOH at 65°C for 20 min. The reaction product was phenol : chloroform purified and precipitated with ammonium-acetate in the presence of PelletPaint (Calbiochem-Novabiochem, Bad Soden, Germany).

Double-stranded T7-tagged cDNA was dissolved in 12 µL H₂O, and 6 µL were used for in vitro transcription with T7 RNA Polymerase Megascript Kit (Ambion, Huntingdon, UK) according to the manufacturer's instructions.

The aRNA was purified using RNeasy columns (Qiagen, Hilden, Germany) and quantified. For each sample, 8 µg of aRNA was reverse transcribed in the presence of I µg random hexamer primer under the same conditions as for the first synthesis (see above). Second-strand strand synthesis was performed as a linear PCR with a 5'-biotin tagged BsmBI oligo-dT18 primer (biotin-GAC ATGCTGCATTGAGACGATTCTTTTTTTTTTTTTTTTTT) and AdvantageTaq 2 (Clontech GmbH; Heidelberg, Germany) according to the manufacturer's instructions.

Double-stranded amplified cDNA was digested with DpnII, 3'DpnII fragments were isolated with streptavidin coupled paramagnetic beads (Dynal Biotech, Hamburg, Germany), and released from the beads with a BsmBI digest. DpnII/BsmBI double stranded cDNA-fragrnents were tagged by cloning the fragments into the TAG-vector (pLCV) which was digested with Bbsl and BamHI. After electroporation of DH10B E. coli (Invitrogen) for each sample 10⁶ independent clones were harvested and the plasmid DNA containing the tagged cDNA was extracted. Using PCR, the tagged cDNAs were amplified from the plasmid DNA and mixed with microbeads (LYNX, Hayward, CA) carrying the complementary antitags. The tagged cDNA was loaded onto the microbeads by hybridizing the tags to the antitags. The DNA loaded beads are loaded into a flow cell and are further processed on an MPSS instrument (LYNX, Hayward, CA) as described (Brenner et al.,(2000) *Nat. Biotechnol.,* 18:630-634).

### Statistics

Statistical significance of the observed signature frequency distributions was calculated using equation (2) from (Audic and Claverie (1997), *Genome. Res.,* 7:986-995). P values smaller than 0.001 were considered significant.

### Quantitative PCR

RNA was isolated according to standard protocols (Chomczynski and Sacchi (1987), *Anal. Biochem.,* 162, 156-159), followed by Qiagen RNeasy™ mini kit purification.
Tissue samples were taken from mouse ipsi- and contralateral to the lesion side 20h after MCAO. cDNA was synthesized from 10 µg total RNA using oligodT primers, superscript II reverse transcriptase (Gibco) using standard conditions. Quantitative PCR was performed using the Lightcycler® system (Roche Diagnostics, Mannheim, Germany) with SYBR-green staining of DNA doublestrands. Cycling conditions were as follows: 10 min 95°C, 5 sec 95°C, 10 sec 65°C, 30 sec 72°C; 10 sec 87°C for 50 cycles. Melting curves were done with the following parameters: 95°C cooling to 50°C; ramping to 99°C at 0.2°C/ sec. The following primer pairs were used: "mm tweak-144s" AAC GCT GTC TGC CCA GGAGCC and "mm tweak-305as" GGC CGA GGA TGA ACC TCA TAA TGG. The Lightcycler® PCR was performed using the SYBR green master mix, following the manufacturer's recommendations (Roche Diagnostics). Specificity of products was ensured by melting point analysis and agarose gel electrophoresis. cDNA content of samples was normalized to the expression level of Cyclophilin (primers: "cyc5" ACC CCA CCG TGT TCT TCG AC; "acyc300" CATTTGCCATGGACAAGATG ). Relative regulation levels were derived after normalization to cyclophilin, and comparison to the sham-operated animal. Error bars indicate standard deviations, these are calculated from 3-fold serially diluted cDNA-samples, and reflect reliability of measurements.

### Example 2: TWEAK efficacy in animal models

TWEAK relevance in stroke can be assessed by using the animal model of middle cerebral artery occlusion in rats (MCAO). In brief, animals receive inhalation anesthesia with 70%N2O, 30% 02, and 1% halothane. A PE-50 polyethylene tube is inserted into the right femoral artery for continuous monitoring of mean arterial blood pressure, and blood gases. The right femoral vein is cannulated by a PE-50 tube for treatment infusion. During the experiment rectal temperature is monitored and maintained at 37 °C by a thermostatically controlled heating pad (Föhr Medical Intruments, Germany). MCAO is induced with a silicon-coated nylon filament that is introduced into the common carotid artery and advanced into the internal carotid artery. Successful MCA occlusion is controlled by Laser-Doppler flowmetry (Perimed 4000, Sweden) with a probe positioned 4 mm posterior to the bregma and 4 mm lateral from the midline. After 90 min occlusion, the filament is withdrawn to allow for reperfusion. Infarct volumes can be calculated by TTC-staining, and computer-assisted planimetry (Image J). Statistical significance is calculated by t-test, and results are considered significant at p<0.05.
TWEAK can be given as an i.v. infusion, or a direct intracranial (e.g. intracerebroventricular) infusion. With this, the predicted negative impact on infarct outcome in vivo can be proven. To demonstrate feasibility of therapeutic intervention with the action of the stroke-acitvated TWEAK, neutralizing antibodies can be given (e.g. intracerebroventricularly).

### Example 3: Setup of a screening system to find novel neuroprotective substances interfering with TWEAK upregulation

It is attractive to screen for novel neuroprotective drugs by exploiting the above described induction of TWEAK by cerebral ischemia. For this, a cell culture system can be set up using cells with neural characteristices (e.g. primary neurons, PC12 cells, SHSY5 cells, and others). This culture can be treated with oxygen-glucose deprivation (OGD), a model for cerebral ischemia in vitro. Induction of TWEAK can be monitored by using quantitative PCR as described above, or reporter constructs with luciferase containing the TWEAK promoter. Expression can also be monitored using an ELISA system. Cells can then be treated with varying concentrations of substances of interest, and screened for a reduction of TWEAK production. Likewise, the expression of the TWEAK receptor can be monitored and screened for.

### Example 4: Determining effects of TWEAK in neural cell culture

In order to monitor the effect of TWEAK activation in cell culture, primary neuronal cultures (or other neural cells) can be assayed for cell death promoting activities of TWEAK, and the TWEAK receptor Fn14. Primary neuronal cultures: Ten to 12 cortices or hippocampi are dissected from rat embryos E18. The tissue is dissociated using 10 mg/ml trypsin, 5 mg/ml EDTA /DNase (Roche diagnostics, Mannheim, Germany) in HBSS (BioWhitakker, Taufkirchen, Germany). The digestion is stopped using four parts neurobasal medium containing 1X B-27 supplement (Invitrogen, Karlsruhe, Germany), 0.5 mM L-glutamine, and 25 µM glutamate. After centrifugation, the pellet is dissolved in 5 ml medium and cells are plated at a density of 250,000 cells per well of a 24-well-plate on glass cover slips coated with poly-L-lysine. For treatment with the NO-donor NOR3 (Sigma), neurons are treated after 21 days in culture with increasing concentrations of NOR-3 for 24 h. TWEAK can then be added in increasing concentrations. Also, antagonists of TWEAK or the TWEAK receptor can be added. As read-outs for neuronal cell death, various methods can be used. For example, PARP cleavage can be assayed using Western blots. Western blots for PARP cleavage: Primary neurons are scraped off the plate and washed twice in ice-cold PBS containing 2.5 mg/ml pepstatin (Sigma-Aldrich, Seelze, Germany) und aprotinin (1:1000, Sigma-Aldrich). Pellets are resuspended in 1 volume 2% SDS (40 µl), and 5 µl benzonase solution (40 µl 100 mM MgCl2 and 9 µl benzonase, Roche Diagnostics, Mannheim, Germany) is added. After solubilization, 1 volume PBS is added and the protein concentration determined (BCA-Test, Pierce, Rockford, IL, USA). After denaturing at 95°C for 5 min, 100 µg is run on 8% SDS-polyacrylamide gels. Proteins are transferred to nitrocellulose membranes (Protan BA79, Schleicher & Schuell, Dassel, Germany) using a semi-dry-blotting chamber (Whatman Biometra, Gottingen, Germany). Blots are blocked with 5% milk powder in PBS/ 0.02% Tween 20, washed three times with PBS/ 0.02% Tween 20, and incubated for 1 h at room temperature with the primary antibody (anti-cleaved-PARP-antibody, Cell Signalling, 1:1000). After washing, the blots are incubated with the secondary antibody (anti-rabbit-antiserum HRP-coupled or anti-mouse-antiserum HRP-coupled, Dianova, Hamburg, Germany 1:4000) for 1 h at room temperature. Signals are detected using the supersignal chemiluminescence system (Pierce, Rockford, USA) and exposed to Hyperfilm-ECL (Amersham Pharmacia Biotech, Piscataway, NJ, USA). PARP cleavage can be quantified on scanned autoradiographs using Windows ImageJ vl.29 (http://rsb.info.nih.gov/ij/index.html).

Alternatively, the cell-death ELISA (Roche diagnostics), LDH assay, the caspase glo assay (Promega), or any other assay used to determine cell death or cell survival can be used that is known to a person skilled in the art.

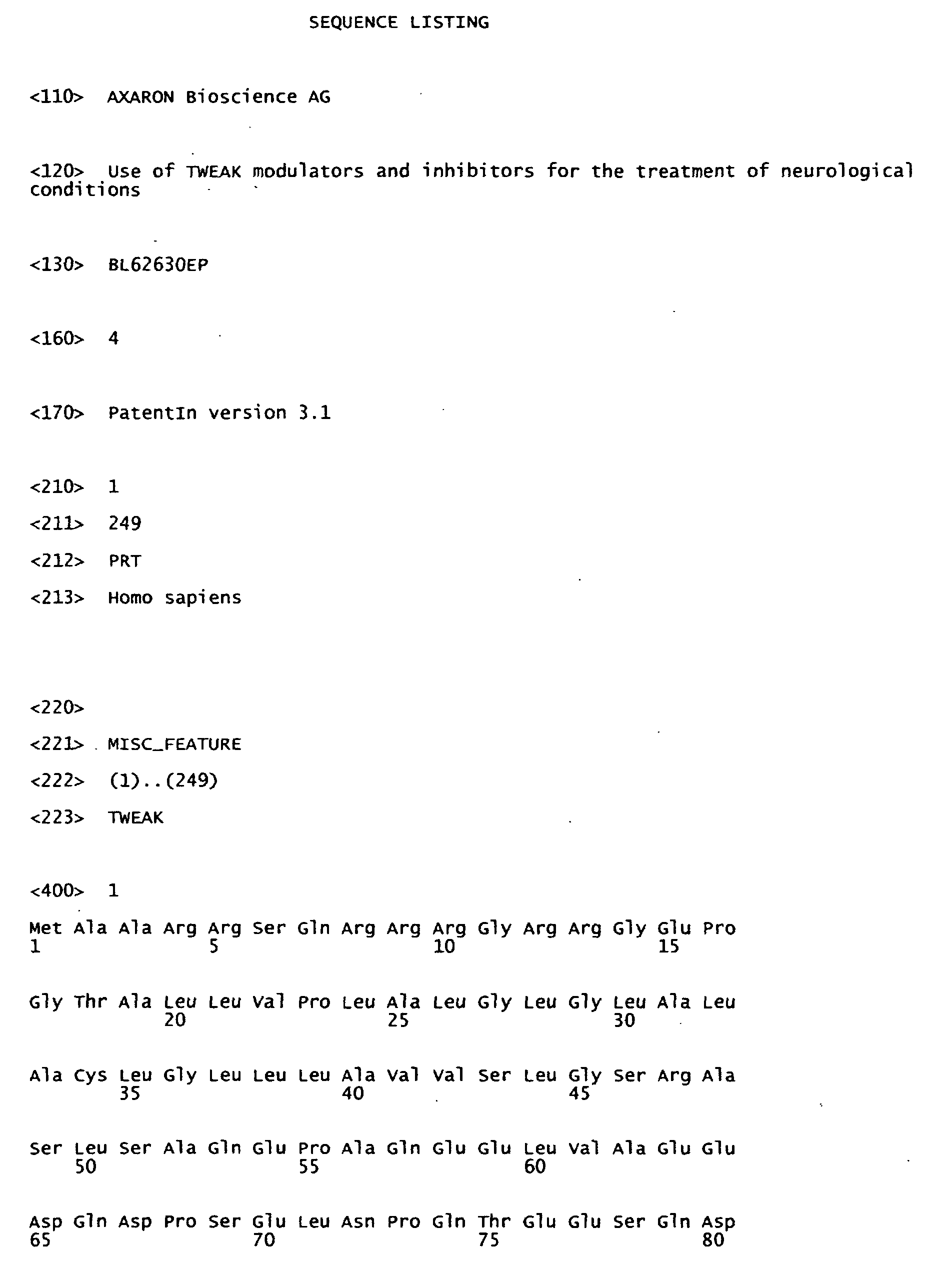

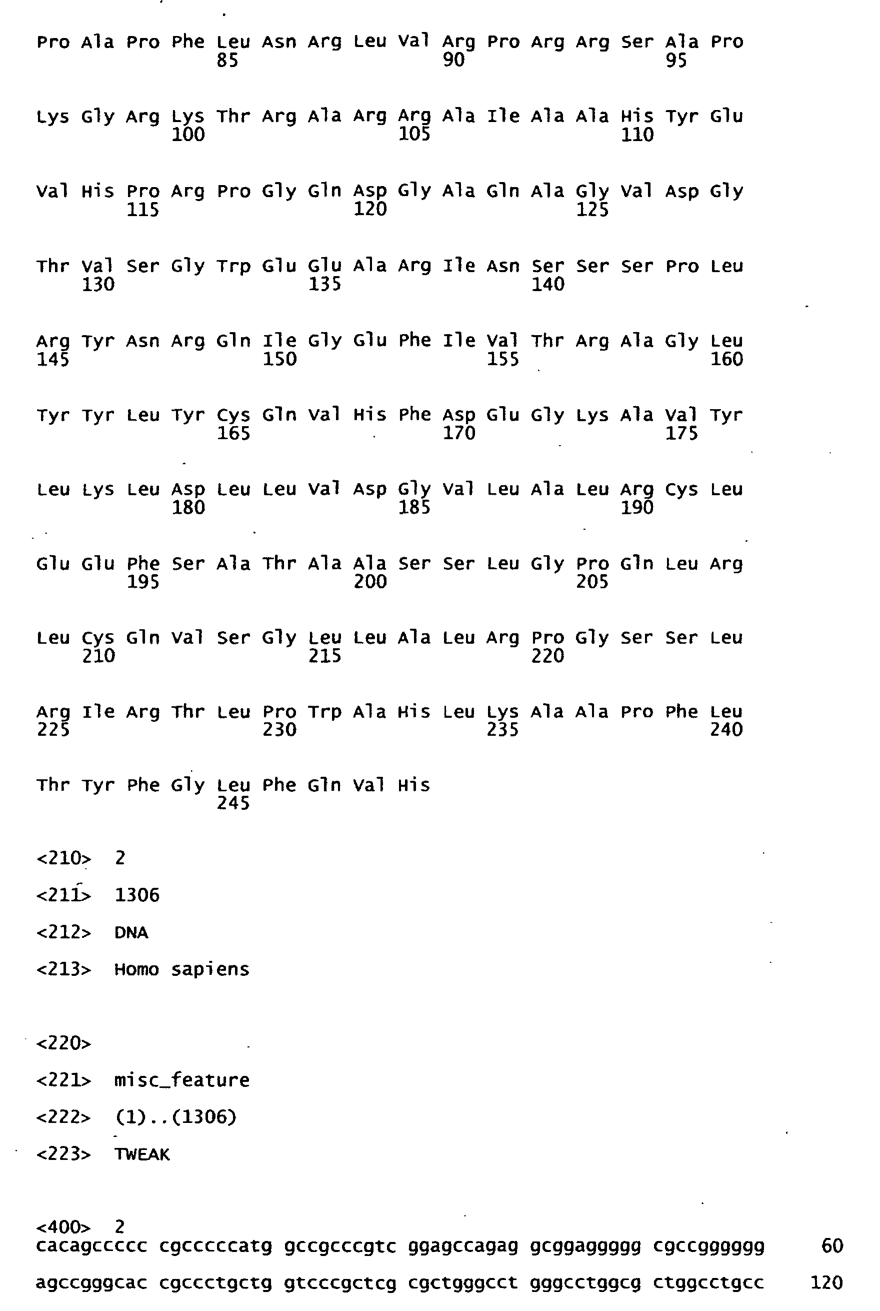

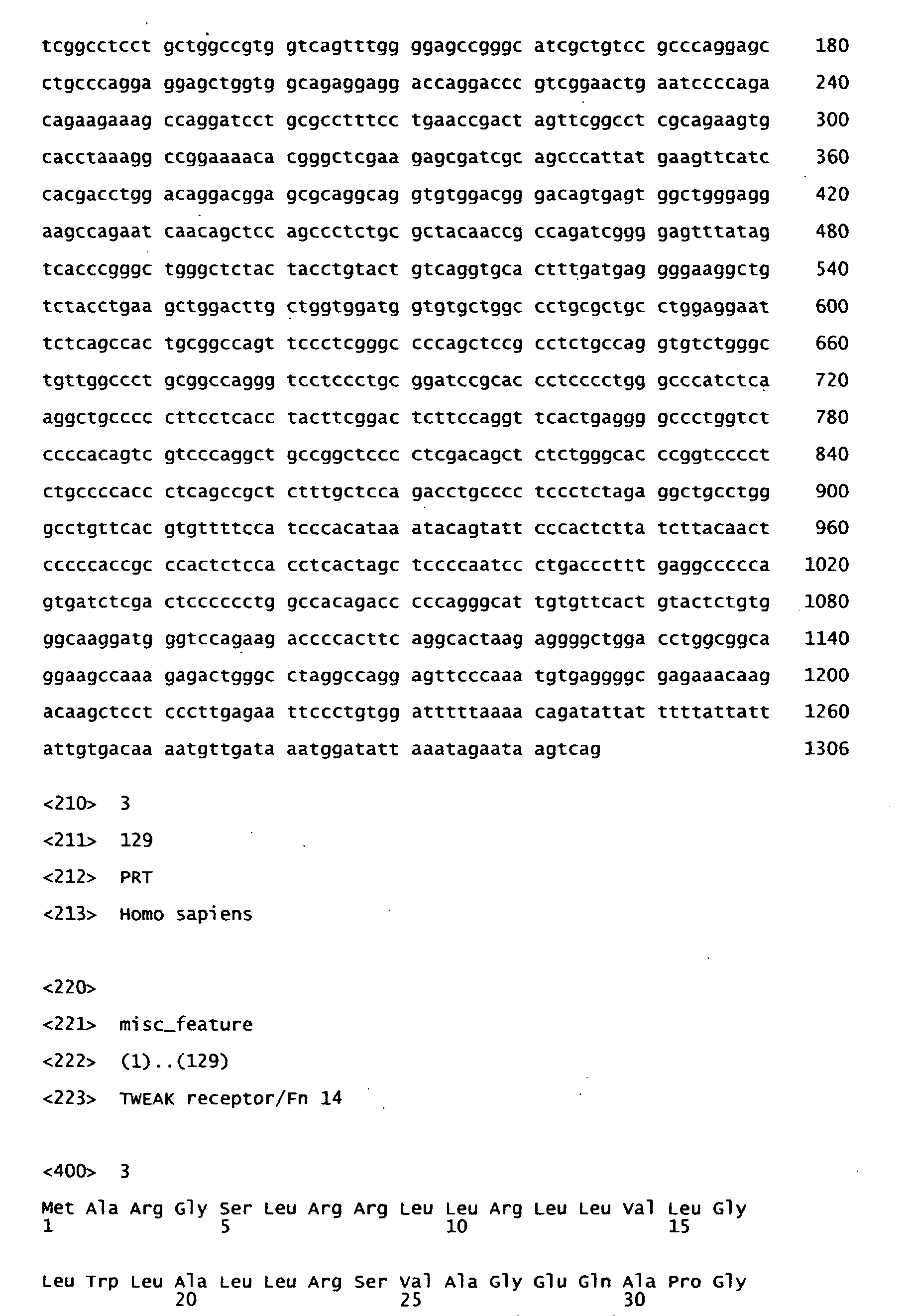

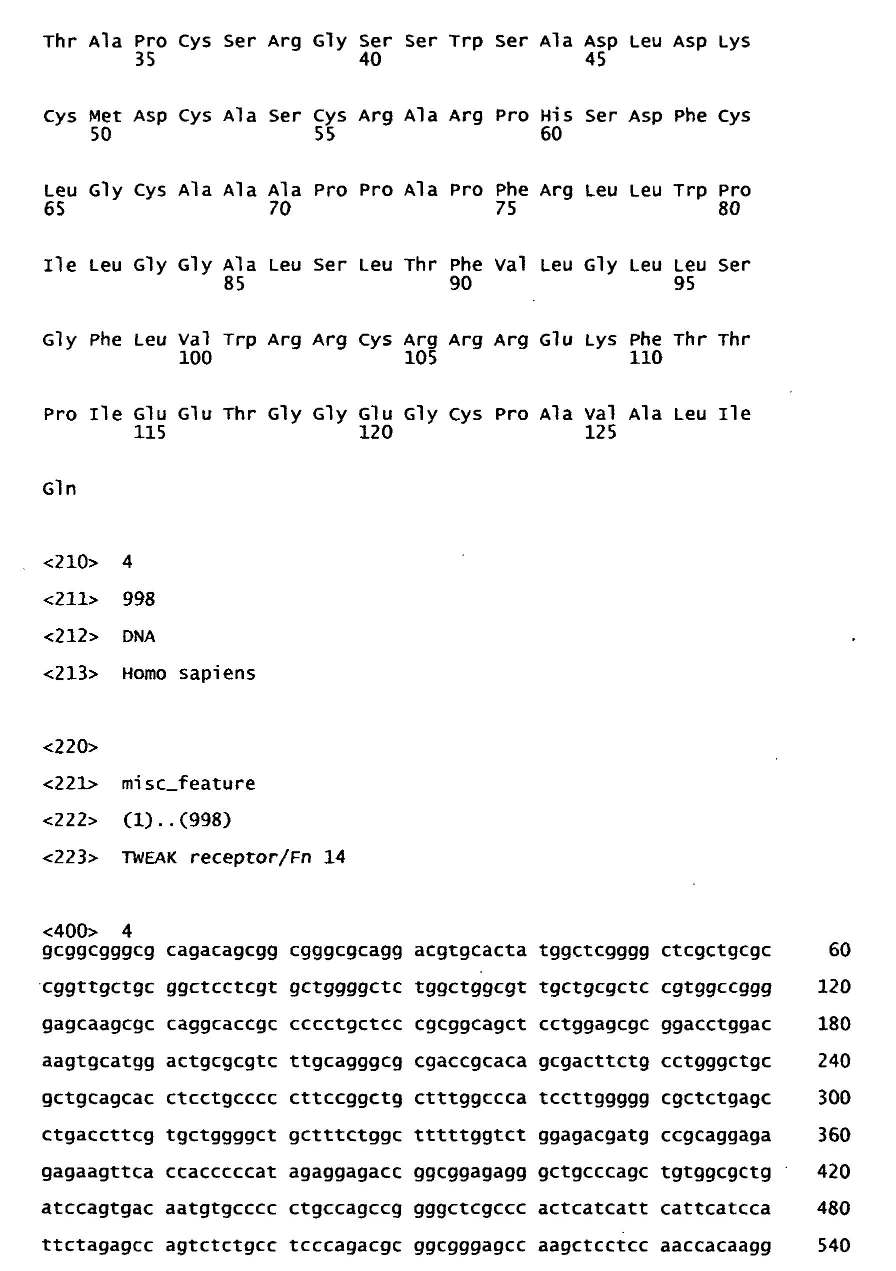

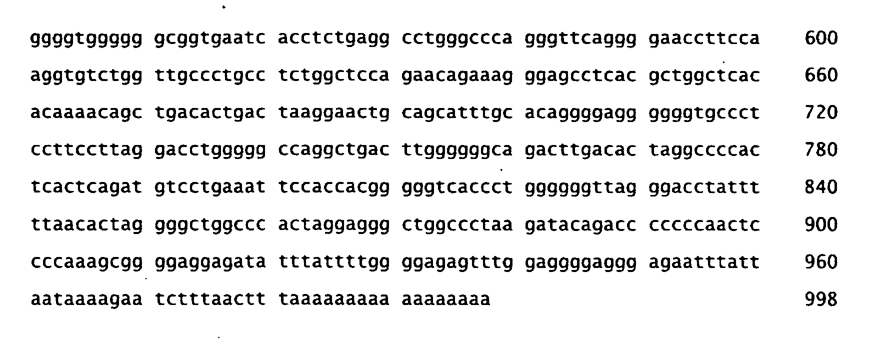

## Claims

1. Use of a substance that modulates or inhibits the expression or activity of TWEAK or of the TWEAK receptor, or of TWEAK and the TWEAK receptor, or that modulates or inhibits the intracellular signalling of the TWEAK receptor, in neural cells, for the manufacture of a medicament for the treatment, or prophylaxis or treatment and prophylaxis of neurological or psychiatric conditions or neurological and psychiatric conditions.

2. The use according to claim 1, wherein the substance is an antibody.

3. The use according to claim 1, wherein the substance is an antisense oligonucleotide.

4. The use according to claim 1, wherein the substance is siRNA.

5. The use according to claim 3 or 4, wherein the substance is provided by a viral vector or a liposome.

6. The use according to claim 1, wherein the substance is a TWEAK-digesting ribozyme.

7. The use according to claim 1, wherein the substance is an antagonist of the TWEAK receptor.

8. The use according to claim 7, wherein the antagonist has a molecular weight of less than 1000 g/mol.

9. The use according to claim 1, wherein the substance is a soluble part of the TWEAK receptor.

10. The use according to claim 1, wherein the neurological condition is at least one condition selected from the group consisting of a neurological disease with pathophysiological mechanisms involving ischemia or hypoxia or ischemia and hypoxia, a neurodegenerative disease, and a disease of the nervous system accompanied by neural cell death.

11. The use according to claim 10, wherein the neurological disease is at least one disease with pathophysiological mechanisms involving ischemia or hypoxia or ischemia and hypoxia selected from the group consisting of stroke, cerebral ischemia after resuscitation, intrapartal hypoxia, intraoperative hypoxia or ischemia or intraoperative hypoxia and ischemia, and intraocular ischemia or hypoxia or intraocular ischemia and hypoxia.

12. The use according to claim 10, wherein the neurodegenerative disease is at least one disease selected from the group consisting of stroke, ALS, Parkinson's disease, cerebral ischemia, multiple sclerosis, schizophrenia, depression, Huntington's disease, trinucleotide repeat disorders, peripheral neuropathies, dementias, and CNS trauma.

13. The use according to claim 10, wherein the disease of the nervous system accompanied by neural cell death is at least one disease selected from the group consisting of stroke, ALS, Parkinson's disease, cerebral ischemia, cerebral ischemia after resuscitation cardiovascular disease, multiple sclerosis, schizophrenia, intrapartal hypoxia, intraoperative hypoxia or ischemia or intraoperative hypoxia and ischemia, intraocular ischemia or hypoxia or intraocular ischemia and hypoxia, depression, Huntington's disease, trinucleotide repeat disorders, peripheral neuropathies, dementias, and CNS trauma.

14. The use according to claim 1, wherein the medicament further comprises one or more additional factors.

15. A method for identifying a substance, that modulates or inhibits the expression or activity of TWEAK, comprising
a) incubating a neural cell culture under conditions that mimic stroke,
b) contacting the cells with the substance,
c) comparing the level of TWEAK expression of these cells with the level of TWEAK expression of cells not contacted with the substance but incubated under the same conditions.

16. A method for the treatment or prophylaxis or treatment and prophylaxis of neurological or psychiatric conditions using a substance, that that modulates or inhibits the expression or activity of TWEAK or of the TWEAK receptor, or of TWEAK and the TWEAK receptor, or that modulates or inhibits the intracellular signalling of the TWEAK receptor, in neural cells.
